# Europäisches Patentamt
## European Patent Office
## Office européen des brevets

(11) Publication number: **0 101 166**
**B1**

(12) **EUROPEAN PATENT SPECIFICATION**

(45) Date of publication of patent specification: 26.08.87

(51) Int. Cl.⁴: **G 01 N 33/571**

(21) Application number: **83303601.5**

(22) Date of filing: **22.06.83**

(54) **Method of measuring infectious disease antibodies.**

(30) Priority: **14.07.82 JP 121244/82**

(43) Date of publication of application:
**22.02.84 Bulletin 84/08**

(45) Publication of the grant of the patent:
**26.08.87 Bulletin 87/35**

(84) Designated Contracting States:
**DE FR GB IT NL**

(56) References cited:
**EP-A-0 038 150**
**EP-A-0 038 181**
**GB-A-1 563 355**
**GB-A-2 045 431**
**US-A-4 184 849**
**US-A-4 313 927**

(73) Proprietor: **FUJIREBIO KABUSHIKI KAISHA also trading as FUJIREBIO INC.**
**6-7, Shimoochiai 4-chome Shinjuku-ku**
**Tokyo 161 (JP)**

(72) Inventor: **Sato, Takashi**
**A-310 20, Nakaarai 3-chome**
**Tokorozawa-shi Saitama-Ken (JP)**
Inventor: **Kubo, Emiko**
**766, Zooshiki 3-chome**
**Higashiyamato-shi Tokyo (JP)**
Inventor: **Kayashima, Takako**
**8-7, Gotenyama 1-chome**
**Musashino-shi Tokyo (JP)**

(74) Representative: **Silverman, Warren et al**
**HASELTINE LAKE & CO. Hazlitt House 28**
**Southampton Buildings Chancery Lane**
**London WC2A 1AT (GB)**

Courier Press, Leamington Spa, England.

**Description**

This invention relates to a method of determining the presence of and/or measuring the level of an infectious disease antibody, specifically TP IgM antibody, to be found in cases of syphilis.

Diagnostic methods for syphilis include the STS (Serologic Test for Syphilis) methods using cardiolipin which is a lipoidal antigen and methods using *Treponema pallidum* (hereinafter referred to as TP) as antigen.

The STS methods, under which fall the VDRL method, the RPR (Rapid Plasma Reagin Card Test) method, the agglutination method, Ogata's method, and Kolmer's method, occasionally indicate a biologically false positive result due to the ability of the test method to respond positively to the presence of an antigen which is not TP antigen.

Insofar as the methods using TP antigen are concerned, the so-called TPI (*Treponema pallidum* Immobilisation) method, FTA-Abs (Fluorescent Treponemal Antibody Absorption) method, and the TPHA (Treponema pallidum Hemagglutination) method have found use. Of these, the TPHA method has been developed so as to allow its automatic operation in the micro titre method, and now it is widely employed as a typical diagnosing method for syphilis.

The erroneous indication of a biologically false positive result of such a TP antigen-using method, such as the TPHA method, is less than that of an STS method. However, this method is not always suitable for use when monitoring the progress of syphilis after treatment in order to judge the success of an attempted cure, because it is responsive to all TP antibodies (see, for example, Yoshio Fukuoka, Nippon Rinsho, vol. 38, p 1501 (1980)). In particular, the conventional method using TP antigen cannot distinguish the IGM produced at the primary stage of syphilis, the IgG produced at its latter stage, and other immunoglobulins.

In 1979, B. Schmidt reported that determination of the stage of syphilis and the necessity of treatment can be made by measuring the syphilis IgM by the SPHA (Solid Phase Hemadsorption) method using anti human IgM antibody and erythrocytes sensitised with TP (Bruno Schmidt, Sex. Trans. Dis., vol. 7, pp 53—58 (1980)). The procedure in this method is simpler than that of the prior method using IgM-FTA-Abs (P. O'Neil, Br. J. Vener. Dis., vol. 48 pp 460—463 (1972)). However, the sensitivity of this method against primary syphilis is low, and the rate of false negative of this method is high.

On the other hand, F. Muller et al separated IgM fraction and IgG fraction from serum by the gel filtration method, and they achieved 19S (IgM)-TPHA and 19S (IgM)-FTA-Abs for each fraction. They judged the stage of syphilis by the results, and used the results in establishing the degree of curing (F. Muller, WHO/VDT/RES., vol. 79, p 361 (1979)). However, this gel filtration method requires a particular apparatus and a long time to carry out, and it utilises large amounts of TPHA reagents. Furthermore, it misses the lower (8S) molecular of IgM which is often found with autoimmune disease, since IgM and IgG are separated from each other according to the differences in their molecular sizes in this method (F. Muller, Klinische Wochenschrift, vol. 57, pp 667—671 (1979)).

Mention may be made finally of GB—A—2045431 which discloses a particle agglutination assay for antibodies and other binding proteins using two different microscopic or submicroscopic particulate reagents. The first particulate reagent binds with the protein under assay and then the second particulate reagent is added which binds only to those first reagent particles which have bound to the protein under assay, so causing agglutination. This procedure serves to amplify the weak agglutination which may be achieved with the first particulate reagent when the binding protein is present in only very small amount. This method is not proof against indicating biologically false positive results. There is no teaching of the application of this method to the determination of the presence or level of anti TP IgM antibodies.

It is an object of this invention to provide a method of measuring an infectious disease antibody, specifically TP IgM antibody, which does not indicate biologically false positive results and does not run the risk of missing the antibody of interest.

According to the present invention there is provided a method of determining the presence or level of TP IgM antibodies in a sample comprising the steps of:

(1) adding first carrier particles having anti IgM antibodies immobilised thereon to a sample liquid thought to contain IgM antibodies to allow the anti IgM antibodies to react with IgM antibodies,

(2) separating the thus treated first carrier particles from the liquid and adding them to a liquid testing medium,

(3) adding second carrier particles having TP antigens immobilised thereon to the liquid test medium containing the first carrier particles to allow the antigens to react with TP IgM antibodies which are present and

(4) observing any agglutination which occurs as indication of the presence on the first carrier particles of TP IgM antibodies and optionally additionally correlating the agglutination due to reaction which has occurred in the preceding step with the level of TP IgM antibodies in the sample liquid.

This invention is based on the discovery that when particular immunoglobulins of a sample are treated with an anti-antibody specific to that type of immunoglobulin sensitised on carrier particles, and the particles are removed from the medium in which contacting has occurred and brought into contact with an antigen of an infectious disease, specifically syphilis, sensitised on carrier particles in a liquid medium, the specific IgM antibody, to the antigen will combine therewith, if present and agglutination will occur.

This invention also provides a kit of materials for use in the measurement of TP IgM antibodies, which comprises anti IgM antibody immobilised on carrier particles, TP antigen immobilised on carrier particles, a diluent and absorbing solutions.

Immunoglobulins are antibodies of an infectious disease, and include IgM, IgG, IgA, IgE and IgD. A sample containing such immunoglobulins is usually the serum of a person to be diagnosed for the infectious disease or a diluted solution of the serum. This sample is pretreated by pH adjustment, by centrifuging or by the addition of a stabiliser, if necessary.

The anti-immunoglobulin antibody to be used is anti IgM antibody which, like anti IgG antibody, is commonly available. These anti antibodies are able to react with infectious disease IgM and IgG antibodies respectively.

The anti-IgM antibody used in carrying out the inventive method may be prepared in a conventional manner. For this purpose, IgM which usually originates from a human source is injected into a warm-blooded animal, such as rabbit, sheep, guinea pig or chicken, and the anti IgM antibody appearing in its blood is collected. The anti-antibody may alternatively be produced as a monoclonal antibody. In this case, IgM is injected into, for example, the abdominal cavity of BALB/C strain mouse, and its spleen is isolated after two weeks. A spleen cell is fused with mouse myeloma P3U1 cell by a conventional method, such as by using polyethylene glycol. The hybridoma thus obtained is cultured and cloned. The cell capable of producing the anti-antibody so produced is injected into abdominal cavity of a mouse, and multiplied. Then, ascites is collected, and the anti-antibody is separated from the ascites.

The anti-antibody is separated from blood or ascites, and purified by a conventional method for separating immunoglobulin, such as precipitation using ammonium sulphate, ion-exchange chromatography using DEAE-cellulose, the gel filtration method, or affinity chromatography.

Such an anti-antibody is immobilised on carrier particles. The carrier particles may be conventional particles for the passive hemagglutination (PHA) method, and include various erythrocytes such as sheep erythrocytes and chicken erythrocytes, microbial cells, polystyrene latex, and gelatin particles (European Published Patent Application No. 0062965). The method for the immobilisation of the anti-antibody on the carrier particles may also be conventional, and methods which may be used include a method using tannic acid, a method using formalin, a method using glutaraldehyde, a method using bisdiazotised benzidine, a method using pyruvic aldehyde and a method using toluene-2,4-diisocyanate.

After the contacting of the carrier particles having anti-IgM antibodies with the sample liquid and allowing the anti-IgM antibodies to react with IgM antibodies, the carrier particles are separated from the solution, and unreacted immunoglobulins, i.e. non-IgM immunoglobulins, are thereby removed. The separation may be carried out by centrifuging or filtering. The recovered carrier particles are preferably washed several times by suspending in water, a saline solution or a buffer solution containing absorbents and effecting vibration, stirring or ultrasonic treatment and recovering the particles by centrifuging or filtering.

The washed carrier particles are suspended in a solution such as a diluent for the PHA method to form a dilution series of the recovered immunoglobulin. Should the immunoglobulin be released from the carrier particles as a result of a particular treatment then it does not matter that at this stage and the dilution series may be regarded rather as constituted by the solutions alone.

TP antigen is then to be added to the dilution series. This antigen is obtained from a culture of TP, such as destroyed TP cells. The carrier particles on which the antigen is sensitised may be conventional for the PHA method, and the sensitisation may also be a conventional variant of the PHA method. Thus, commercially available TP sensitised carrier particles may be employed as the antigen in the method of the present invention.

The TP antigen sensitised on carrier particles is contacted with the dilution series of the recovered immunoglobulin. The contacting may be carried out by the conventional procedure of the PHA method. For this purpose, the antigen sensitised on carrier particles is suspended in a reconstituting solution, and added to the dilution series of the recovered immunoglobulin. Then, the mixture is incubated at 4 to 40°C, usually at ambient temperature, for 30 minutes to 25 hours, and the pattern of the carrier particles in each well is observed to see whether or not agglutination occurs.

The reagents required when carrying out the method of this invention other than the antigen immobilised on carrier particles and the anti-immunoglobulin antibody immobilised on carrier particles are absorbing solutions and a diluent. Insofar as the absorbing solutions are concerned, two kinds are necessary. One is for the antigen immobilised on carrier particles, and the other is for the anti-immunoglobulin antibody immobilised on carrier particles. In general, a standard serum, unsensitised carrier particles and a reconstituting solution are usually employed. These reagents may be the same as employed in the PHA method.

The method of the invention possesses superior specificity. When carrying out this method, it is possible for a specific antibody of a specifically infectious diesease, namely syphilis IgM, to be simply and rapidly measured by using usual equipment. The data obtained by the method of the invention are useful for judging the stage of the infectious disease, monitoring the result of treatment, and evaluation of the necessity of further treatment.

The present invention is further illustrated by the following examples.

3

# 0 101 166

Example 1

(i) Preparation of TP antigen

The WHO pathogenic standard strain, Treponema pallidum Nichols, obtained from the National Institute of Health, Ministry of Health & Welfare (Japan) was cultured, and the multiplied cells were suspended at a concentration of $6.0 \times 10^7$ cells/ml.

One ml of the suspension was inoculated in each testes of each of 10 rabbits, and cultured for 12 days. The testes were excised from each rabbit and minced. Multiplied cells were washed out from the minced testes by using 1000 ml of a mixed solution consisting of equal volumes of inactivated normal rabbit serum and 1/7 M saline solution.

The extracts thus produced were centrifuged at $200 \times g$ for 10 minutes, and the precipitated material was removed. The supernatant was centrifuged again at $19,000 \times g$ for 90 minutes, to precipitate thereby the TP cells.

The precipitates containing the TP cells were washed three times with chilled 0.075 M sodium oxalate, and were suspended in 10 ml of 1/15 M phosphate buffer solution having a pH of 6.4. The number of TP cells in the suspension was counted, and then the suspension was diluted to obtain a TP antigen solution in which the concentration of the TP cells was $2 \times 10^9$/ml.

13 ml of 17 w/v% of sodium diatrizoate solution were placed in a 14 ml cellulose tube for ultracentrifugation, and one ml of the TP antigen solution was layered on it. The cellulose tube was placed in a horizontal rotor SW40Ti of a centrifuge (Beckman L8), and centrifuged at 4°C at 23,500 rpm ($100,000 \times g$) for 45 minutes. The pelleted TP cell material at the bottom was collected, and suspended in 8 ml of a phosphate buffered saline solution (PBS) of pH 7.2. The TP cells were destroyed by a homogeniser (Tomy Seiko UR—200P, Tokyo, Japan), and preserved at −80°C.

(ii) Preparation of TP antigen sensitised erythrocytes

20 ml of 2.5 v/v% formalinised sheep erythrocytes suspension in PBS (pH 7.2) were mixed with 20 ml of 10 ppm tannic acid PBS (pH 7.2) solution, and incubated at 37°C for 10 minutes. The erythrocytes were collected by centrifugation, and washed with a saline solution. The washed erythrocytes were suspended in PBS (pH 6.4) to 20 ml.

The already prepared TP antigen solution was diluted 80 times, and 20 ml of the diluted TP antigen solution were mixed with 20 ml of the above washed erythrocytes suspension. The mixture was incubated at 37°C for 40 minutes with stirring at intervals of 10 minutes.

The erythrocytes were collected by centrifuging, and washed with a saline solution containing 1% normal rabbit serum. The erythrocytes were suspended in PBS (pH 7.2) containing 1% normal rabbit serum to 10 ml, and each 0.5 ml of the suspension was pipetted into a 5 ml vial. Then, the erythrocytes in each vial were lyophilised, and preserved at 4°C.

(iii) Preparation of anti human immunoglobulin antibody sensitised erythrocytes

Sheep erythrocytes were treated with tannic acid in the manner indicated in the foregoing section (ii).

20 ml of the suspension of sheep erythrocytes treated with tannic acid suspended in PBS (pH 6.4) were mixed with 20 ml of rabbit immunoglobulins to human IgM antibody µ-chain specific, Dako) diluted 400 times, and the mixture was treated by the procedure set out under section (ii) to produce a lyophilised anti human IgM antibody sensitised erythrocytes.

(iv) Hemagglutination immunoassay (HIA)

One ml of distilled water was added to each of the above-indicated lyophilised products, i.e., anti human IgM antibody sensitised erythrocytes (AM-RBC), and TP antigen sensitised erythrocytes (TP-RBC), to achieve reconstitution thereof.

5 µl of a sample serum were placed in a left end well (well No. 1) of a micro titre plate, and 50 µl of B-solution (a combined solution of an absorbing solution with a diluent which is one of the components of the kit for TPHA test, manufactured by Fujirebio Inc., Japan) were added to the contents of the same well. A further 25 µl of the B-solution were placed in each remaining well, i.e. the wells extending from the second well (well No. 2) from the left end to the well at the right end of the plate. Then, the sample serum in the well No. 1 was diluted by using a diluter, and a twice by twice dilution series of the sample serum was prepared.

The reconstituted AM-RBC suspension was diluted with 7 ml of the B-solution the suspension then corresponding to an 8 times diluted suspension of the original 2.5% AM-RBC suspension, and 25 µl of the diluted AM-RBC suspension were added to each well from the third well (well No. 3) from the left end to the right end well. 25 µl of unsensitised blood cell (RBC) suspension were added to well No. 2.

The mixture in each well was sufficiently mixed by vibrating of the micro titre plate, and the plate was covered by a glass plate. The plate was then incubated at 37°C for 15 minutes, and the mixtures in the wells were centrifuged at ambient temperature at 2,000 rpm ($600 \times g$) for 5 minutes. The supernatant in each well was drawn off by suction and 25 µl of saline were added to each well. The blood cells in each well were suspended by vibrating the micro titre plate, and centrifuged at 2000 rpm ($600 \times g$) for 5 minutes. Then, the supernatant in each well was again drawn off by suction.

25 µl of the B-solution were added to each well, and the erythrocytes were suspended by vibrating. 25 µl of the reconstituted TP-RBC suspension were added to each well from well No. 3 to the right end well. 25

μl of the RBC suspension were added to well No. 2. The mixture in each well was sufficiently stirred, and allowed to stand for 2 hours or overnight. Then, the pattern of erythrocytes in each well was observed to see whether or not hemagglutination had occurred.

The antibody titre as determined is expressed in the dilution ratio of serum, and an antibody titre greater than 40 is indicated as positive.

The procedure employed for the dilution is shown in the following Table 1.

TABLE 1

| Well No. | 1 | 2 | 3 | 4 | 5 | 6 | . . . . . . . |
|---|---|---|---|---|---|---|---|
| Dilution ratio of serum | 10 | 20 | 40 | 80 | 160 | 320 | . . . . . . . |
| Serum | 5 | 25 | 25 | 25 | 25 | 25 | . . . . . . . |
| B-solution | 50 | 25 | 25 | 25 | 25 | 25 | . . . . . . . |
| AM-RBC | — | — | 25 | 25 | 25 | 25 | . . . . . . . |
| RBC | — | 25 | — | — | — | — | — |
| B-solution | — | 25 | 25 | 25 | 25 | 25 | . . . . . . . |
| TP-RBC | — | — | 25 | 25 | 25 | 25 | . . . . . . . |
| RBC | — | 25 | — | — | — | — | — |

(v) Practical measurements

Determinations were carried out using 3 sample sera of patients with primary syphilis, 3 sample sera of patients with secondary syphilis, 3 sample sera of patients with late latent syphilis and 5 sample sera of healthy persons. The methods employed were those of the above section (iv) (HIA), the TPHA method (using reagents etc. for the purpose manufactured by Fujirebio Inc), the FTA-Abs method (Pub. Health Rep., vol. 79, pp 410—412 (1964)), the RPR method (Pub. Health Rep., vol. 77, pp 645—652 (1962)), Ogata's method (Nisshin Igaku, vol. 47, pp 671—689 (1960) and the gel filtration-TPHA method (Nippon Iji Shinpo, No. 3002, pp 43—47 (1981)).

The results obtained are shown in the following Table 2.

5

TABLE 2

| Stage | Serum | HIA | | | TPHA | | FTA-Abs | | RPR | Ogata | Gel filtration -TPHA | |
|---|---|---|---|---|---|---|---|---|---|---|---|---|
| | | IgM | IgA | IgG | Old | New | μ | μ+γ | | | 19S (IgM) -TPHA | 17S (IgG) -TPHA |
| Primary | H.A 8/12 | 160 | 80 | (−) | 20ʷ | 320 | 20 | 20 | 1 | 10 | 2.1 | 0 |
| | T.A 2/16 | 640 | 1280ʷ | (−) | 80 | 640 | 20 | 80 | 16 | 160 | 10.2 | 0 |
| | T.K 1/8 | 320 | 320 | (−) | 40 | 640 | 20 | 20 | 16 | 320 | 5.1 | 0 |
| Secondary | S.K 12/2 | 320 | 1280ʷ | 2560ʷ | 1280 | 2560 | 20 | 1280 | 128 | 640 ↑ | 6.5 | 30.1 |
| | Y.N 11/5 | 2560 | 1280 | 640 | 1280 | 2560 | 20 | 1280 | 128 | 1280 | 15.1 | 12.9 |
| | S.N 7/22 | 2560ʷ | 1280 | 1280 | 1280 | 2560 | 5 | 1280 | 128 | 1280 | 13.3 | 16.6 |
| Late latent | H.Y 2/1 | (−) | (−) | 160 | 320 | 160 | (−) | 320 | 1 | 5 | 0 | 2.5 |
| | A.Y 4/3 | (−) | (−) | 160 | 320 | 160 | (±) | 80 | 4 | 10 | 0 | 2.3 |
| | T.K 5/20 | (−) | (−) | 640ʷ | 320 | 160 | (−) | 20 | (ND) | 80 | 0 | 4.7 |

(−) Nonreactive
(±) Borderline
ʷ weak
640 ↑ Greater than 640 times
(ND) Not Determined
γ: IgG, γ-chains specific
μ: IgM, μ-chains specific

As can be seen from Table 2, the results of the present method of this invention (HIA) are substantially identical with those of the gel filtration method which has hitherto been regarded as the most reliable method for determining IgM and IgG.

Example 2

IgM in the sera of a primary syphilis and a late latent syphilis were measured by the HIA method using AM-RBC and TP-RBC.

A washing process using saline solution was carried out once or twice after the reaction with the anti-immunoglobulin antibody sensitised on carrier particles, and the measuring procedures were the same as the section (iv) of Example 1.

The results obtained are shown in the following Table 3.

TABLE 3

| Serum | Number of washings | Antibody titre |
|---|---|---|
| Primary }<br>Syphilis | 1 | 1:1280 |
| | 2 | 1:1280 |
| Late latent }<br>Syphilis | 1 | (−) |
| | 2 | (−) |

As can be seen from the data, the antibody titre was not reduced by washing.

**Claims**

1. A method of determining the presence or level of *Treponema pallidum* (TP) IgM antibodies in a sample comprising the steps of:

(1) adding first carrier particles having anti IgM antibodies immobilised thereon to a sample liquid thought to contain IgM antibodies to allow the anti IgM antibodies to react with IgM antibodies,

(2) separating the thus treated first carrier particles from the liquid and adding them to a liquid testing medium,

(3) adding second carrier particles having TP antigens immobilised thereon to the liquid test medium containing the first carrier particles to allow the antigens to react with TP IgM antibodies which are present and

(4) observing any agglutination which occurs as indication of the presence on the first carrier particles of TP IgM antibodies and optionally additionally correlating the agglutination due to reaction which has occurred in the preceding step with the level of TP IgM antibodies in the sample liquid.

2. A method as claimed in claim 1, wherein the carrier particles are selected from erythrocytes, microbial cells, polystyrene latex particles and gelatin particles.

3. A method as claimed in any one of the preceding claims, wherein the anti-antibody is immobilised on the carrier particles by using tannic acid, formalin, glutaraldehyde, bisdiazotised benzidine, pyruvic aldehyde or toluene-2,4-diisocyanate.

4. A kit of materials for use in the measurement of TP IgM antibodies, which comprise anti-IgM antibody immobilised on carrier particles, TP antigen immobilised on carrier particles, a diluent and absorbing solutions.

**Patentansprüche**

1. Verfahren zur Bestimmung der Anwesenheit oder der Menge von *Treponema pallidum* (TP) IgM-Antikörpern in einer Probe, umfassend die folgenden Stufen:

(1) Zugabe von ersten Trägerteilchen, die mit darauf immobilisierten Anti-IgM-Antikörpern, versehen sind, zu einer Probeflüssigkeit, von der man annimmt, daß sie IgM-Antikörper enthält, zur Herbeiführung einer Reaktion der Anti-IgM-Antikörper mit den IgM-Antikörpern,

(2) Abtrennen der so behandelten ersten Trägerteilchen von der Flüssigkeit und Zugabe derselben zu einem flüssigen Testmedium,

(3) Zugabe von zweiten Trägerteilchen, die mit darauf immobilierten TP-Antigenen versehen sind, zu dem flüssigen Testmedium mit den darin enthaltenen ersten Trägerteilchen zur Herbeiführung einer Reaktion der Antigene mit anwesenden TP-IgM-Antikörpern und

(4) Ermittlung irgendeiner Agglutination, die eintritt als Anzeichen für die Anwesenheit von TP-IgM-Antikörpern auf den ersten Trägerteilchen und eventuell zusätzlicher Vergleich der in der vorhergehenden Stufe durch die Reaktion bewirkten Agglutination mit der Menge der TP-IgM-Antikörper in der Probeflüssigkeit.

2. Verfahren nach Anspruch 1, bei dem die Trägerteilchen ausgewählt sind aus Erythrozyten, mikrobischen Zellen, Polystyrol-Latexteilchen und Gelatineteilchen.

3. Verfahren nach irgendeinem der vorangehenden Ansprüche, bei dem die Anti-Antikörper auf den Trägerteilchen immobilisiert sind durch Verwendung von Tannin, Formalin, Glutaraldehyd, bisdiazotiertem Benzidin, Methylglyoxal oder Toluol-2,2-diisocyanat.

4. Ausrüstungssatz zur Verwendung bei der Bestimmung von TP-IgM-Antikörpern, enthalten auf Trägerteilchen immobilisierte Anti-IgM-Antikörper, auf Trägerteilchen immobilisiertes TP-Antigen, ein Verdünnungsmittel und Absorptionslösungen.

## Revendications

1. Procédé de détermination de la présence ou du taux d'anticorps IgM de *Treponema pallidum* (TP) dans un échantillon, comprenant les étapes qui consistent à:

(1) ajouter des premières particules de support, sur lesquelles sont immobilisés des anticorps anti-IgM, à un échantillon liquide censé contenir des anticorps IgM pour permettre aux anticorps anti-IgM de réagir avec les anticorps IgM,

(2) séparer les premières particules de support ainsi traitées du liquide et les ajouter à un milieu d'essai liguide,

(3) ajouter des deuxièmes particules de support, sur lesquelles sont immobilisés des antigènes TP, au milieu d'essai liquide contenant les premières particules de support pour permettre aux antigènes de réagir avec les anticorps IgM de TP qui sont présents et

(4) observer toute agglutination se produisant comme une indication de la présence, sur les premières particules de support, d'anticorps IgM de TP et établir éventuellement la corrélation entre l'agglutination due à la réaction qui a eu lieu dans l'étape précédente avec le taux d'anticorps IgM de TP dans l'échantillon liquide.

2. Procédé selon la revendication 1, dans lequel les particules de support sont choisies parmi des érythrocytes, des cellules microbiennes, des particules de latex de polystyrène et des particules de gélatine.

3. Procédé selon l'une quelconque des revendications précédentes, dans lequel l'anti-anticorps est immobilisé sur les particules de support au moyen d'acide tannique, de formaline, de glutaraldéhyde, de benzidine bisdiazotée, d'aldéhyde pyruvique ou de toluène-2,4-diisocyanate.

4. Trousse de produits utilisables pour la mesure des anticorps IgM de TP, qui comprend un anticorps anti-IgM immobilisé sur des particules de support, un antigène TP immobilisé sur des particules de support, un diluant et des solutions absorbantes.